# EUROPEAN PATENT APPLICATION

(11) **EP 1 238 630 A2**
(43) Date of publication of application: **11.09.2002**
(21) Application number: 02004183.6
(22) Date of filing: 26.02.2002
(51) Int. Cl.: A61B 5/053, A61B 5/00, A61N 1/365

(54) **Process and implantable device for the intrapulmonary assessing of density dependant physical properties of the lung tissue**

(30) Priority: 01.03.2001 IT BO010110
(71) Applicant: Tre Esse Progettazione Biomedica S.r.l, 40138 Bologna (IT)
(72) Inventor: Plicchi, Gianni, 40136 Bologna (IT); Marcelli, Emanuela, 62100 Macerata (IT)
(74) Representative: Porsia, Dino, Dr.

(57) **Abstract**

The invention relates to an implantable device for therapeutic and diagnostic purposes, which is capable to detect the physical properties of the pulmonary tissue which depend from its density and their variations caused by a pathologic condition of the heart (heart failure, ischemia, arrhythmia), using one or more intrapulmonary catheters, provided with sensors, inserted in branches of the pulmonary artery. The catheters provided with sensors preferably use as sensors electrodes and the detected physical property, which depends by the density of the pulmonary tissue, is preferably the bioelectric impedance of the explored portion of lung. The same electrodes can be also used for monitoring of an electrocardiogram, without artefacts and interferences. The device can be used to guide and optimise the therapy of the heart disease made with electrostimulators and/or cardiac defibrillator and/or systems for the exhibition of drugs and/or for the drainage of the fluids accumulated in the body and/or for the circulatory assistance to the heart. The device comprises means for the communication with external devices by means of radio-frequency transmission. This invention constitutes a significative improvement in the state of the art of the implantable devices for the therapy and the diagnosis of the heart diseases, because it allows the measurement of the specific bioelectric impedance of the pulmonary tissue only, overcoming the drawbacks of the known systems, which by using surfaces electrodes placed upon the skin or electrodes placed inside of the heart cavities, give a measurement of the transthoracic impedance determined prevalently by the volume variations of the heart and great vessels and only partially of the contents of fluids in the pulmonary tissue.

## Description

Heart failure is a serious pathologic condition of the heart, during which the amount of blood pumped by the heart is insufficient to satisfy the normal requirements of oxygen and nutrients of the body. Heart failure has several causes and is a complication of several diseases. Heart failure is more common in the older people because they are more predisposed to the primary pathologies which can be the cause of the same. Heart failure affects about 22,5 millions of persons world-wide and about 2 millions of new cases are diagnosed every year. Five millions of persons are affected by heart failure in the U.S.A., about six millions and half in Europe and about seven hundred and fifty-thousand in Italy. About the 70% of the patients with heart failure die because of said disease within ten years.

Each disease which affects the heart and which interferes with the circulation of the blood, can lead to heart failure. By far the most common disease which can lead to the heart failure is represented by the coronary disease, which reduces the blood flow to the heart and produces an infarct which can damage the cardiac muscle. This disease is predisposed by diabetes, hyperthyroidism or obesity. Diseases of the cardiac valves may obstruct the flow between the chambers of the heart and the aorta and in contrast, a defective valve produces a retrograde haematic flow. These situations increase the workload of the heart which can dilate and reduce its functionality.

Other diseases can affect the electric conduction system of the heart and may cause alteration of the rhythm, which can lead to a myocardial hypertrophy, like any muscle after months of exercise. Initially this dilatation allows a more strong contraction, but a further dilatation may lead to a reduced contractile capacity and to a heart failure.

Also hypertension and the stenosis may increase the heart workload and predispose to heart failure. Human body has physiological responses to compensate the heart failure. First are hormonal responses which cause the release of adrenaline and noradrenaline which improves the contractility of the muscle. Another corrective mechanism is the salt retention by means of the kidneys, which causes a parallel retention of water in the tissues and said fluid increases the amount of circulating blood and improves the cardiac function stretching the heart cavities.

In the failure, the fluids excess permeates the blood vessels and accumulates in several portions of the body causing oedemas.

Although the failure of a single portion of the heart causes damages to the whole heart, the symptoms of the failure of the left portion of the heart lead to a fluid retention in the pulmonary tissue (pulmonary oedema) which causes dyspnea, initially only during the physical exertion, but subsequently, as the disease progresses, it also occurs at rest.

An acute increase of the fluid in the pulmonary tissues (acute pulmonary oedema) is a life-threatening emergency.

During the first stages of the pulmonary oedema, the fluid begins to accumulate in the perivascular tissues and peribronchial pulmonary, where it forms cuffs of fluid around the small airways and the haematic vessels. When the resorption of this fluid made by means of the capillaries and the removal made by means of the lymphatic system is blocked and reduced, the alveolar oedema and the widening of the airways occurs, with consequences which can cause also death.

It is in the early phase of the process that physical and biological factors which normally regulate the volume of interstitial fluid in the pulmonary tissue, the hydrostatic pressure, the permeability and the lymphatic fluid, are the most effective to return the lung to its normal state. Because mild and moderate interstitial oedema is an early indication of the loss of balance between inlet and outlet of fluids in the lung and because in this phase is easily reversible, it is important to detect its occurrence and to monitor with continuity its progress or its resolution.

It would be opportune to have a diagnostic technique which can detect the pulmonary oedema also in the precocious phase, but unfortunately the diagnostic method nowadays available not allow this. To detect early the onset of the pulmonary oedema, it is necessary to measure the physical properties of the pulmonary tissue which are affected by the presence of small quantity of fluid which fills and dilates the interstices.

The methods which are nowadays available to detect the accumulation of fluids inside the pulmonary tissue can be invasive or non invasive.

The invasive methods consist essentially in the biopsy of the pulmonary tissue and are sensibly specific and precise.

The non invasive methods like the radiology and in general the imaging techniques, are not able to distinguish the earlier phases of the oedema from the normal variations from subject to subject.

The bioelectric impedance is one of the physical properties which depends also by the blood and fluids content in the lungs and in the past many attempts have been made to utilise the impedance measured with electrodes placed upon the chest of the body, as a non invasive method for the early diagnosis and for the quantification of the pulmonary oedema and the heart failure.

In the US patent 5.788.643 (Process for monitoring patients with chronic congestive heart failure) for example, is described a system for the monitoring of patients with chronic heart failure, by means of the use of cutaneous electrodes applied on the chest. According to the measured currents and impedances, the indexes of CHF (Congestive Heart Failure) are calculated and the therapeutic intervention begins when the differences between the calculated CHF indexes and the baseline values are outside from the established tolerances.

An important limitation of the above mentioned method is due to the fact that the measurement of the transthoracic impedance is determined principally by the heart and big vessels volume which engage over the 75% of the total of the fluids contained inside the chest, while is determined only partially by the contents of fluid in the lungs. The external measure of the transthoracic impedance can be also affected by artefacts due to the movements of the body.

Several methods and implantable devices have been developed for the measurement of the impedance in the rate-responsive pacemakers, using as electrodes the stimulation standard, for example in the US patent 6.044.294 (Method and apparatus for measuring the impedance in the body).

In the US patents 5.876.353 and 5.957.861 (Impedance monitor for discerning oedema through evaluation of respiratory rate) for example, to detect the oedema is described an implantable monitoring device for the impedance assessing the respiratory rate, where the configuration of the electrodes for the measurement of the impedance can comprise a standard stimulation electrode inserted in the heart and one electrode on the surface of the implantable device. The limit of this method consists in the fact that the respiratory rate can be influenced also by several physiologic conditions of the patient, such as for example the anxious and panic conditions not correlated with the oedema, so that this same method can not detect with well-founded certainty the presence of the pulmonary oedema.

In the US patent 6.104.949 (Medical device) for example, is described an implantable device to determine the degree of the heart failure, which detects both the transthoracic impedance, and the posture of the patient, where the transthoracic impedance can be measured between a standard stimulation electrode, placed in the heart, and a second sub-cutaneous electrode. The limits of this system are in the measurement of the intracardiac impedance, with the limitations above described, to be determined in a prevalent manner by the volume of the heart and of the big vessels, to which the posture sensor can not give response.

In the article "Feasibility of monitoring thoracic congestion with impedance measured from an ICD leas system in a chronic heart failure dog model" by L. Wang et al. PACE vol. 23 (Part II): 612, 2000, for example, is described experimentally a method for monitoring the thoracic congestion, in a model of CHF on the dog, with the utilisation of the impedance measured by means of an electrocatheter of an implantable defibrillator.

Also in this case, as happens with the utilisation of surface electrodes for the detection of the transthoracic impedance, the variations of the biolectric impedance detected with the utilisation of stimulation or defibrillation catheters, are mainly determined by the heart and big vessels volume and only in part by the content of fluids inside the lungs.

Moreover, in patients with heart failure, are revealed important dilatations of the volume of the heart and for this reason in the above mentioned systems is more difficult to separate the variation of the impedance due to the pulmonary density and the variation due to the heart.

In the article "Peribronchial electrical admittance measures lung edema and congestion in dog" by J. E. McNamee and M. L Brodman, J. Appl. Physiol. 49(2): 337-341, 1980, the oedema and congestion in the dog are detected, before the invasion of the fluid inside the alveoli, using a stimulation standard temporarly electrode inserted through the thrachea for the measurement of the impedance of the local tissue near the peribronchial space. It has been demonstrated how the bioelectric impedance can be a sensible index of the content of extravascular fluids in the lung in the examined region.

This method differs from the transthoracic impedance measurement with cutaneous or intracardiac electrodes, because realises a direct contact with the lung, but it is not suitable for a continuous monitoring of the bioelectric impedance, because an electrocatheter can not chronically implanted in the peribronchial space through the trachea and it is not possible an insertion of said electrode directly in the pulmonary tissue with a surgical operation.

Another important limitation connected to the utilisation of stimulation or defibrillation standard electrocatethers inserted in the heart for the measurement of the bioelectric impedance, is represented by the fact that from said electrodes it is possible to register only a local electrogram in the contact zone with the endocardium, not suitable do describe the whole electric cardiac cycle, like happens when are used surface electrodes.

Recently have been developed subcutaneous implantable device for the monitoring of the ECG, like described, for example in the US patent 5.987.352 (Minimally invasive implantable device for monitoring physiological events) but the registrations of the ECG may be affected by artefacts due to the movement or to muscular contractions and connected myoelectric signals.

The invention proposes an apparatus and a method for the detection of the physical properties of the pulmonary tissue which are depending from its density and their variations caused by a pathologic condition (heart failure, ischemia, arrhythmia), using one or more intrapulmonary catheters provided with sensors, inserted through the intravenous way and then through the heart, inside branches of the pulmonary artery. Preferably the intrapulmonary catheters are inserted in the same lung and preferably in the right lung which is less involved by the volume of the heart, so that the work of the same catheters result less disturbed by the movements of the heart during the cardiac cycle.

According to possible realisations of the invention, the intrapulmonary catheters provided with sensors may use thermal flow sensors because the accumulation of fluids modify the thermal conductivity of the pulmonary tissue, as described, for example, in "Assessment of cardiac preload and extravascular lung water by single transpulmonary thermodilution" SAKKA S. G. et al. Intensive Care Med. 2000 Feb; 26(2):180-7, or there can be used optical sensors because the accumulation of fluids modify the trans-luminescence and/or the colour of the pulmonary tissue, as described for example in "Hear disease" E. BRAUNWALD, or may use, for example ultrasonic sensors, because the accumulation of fluids modify the propagation rate of the ultrasounds in the pulmonary tissue, as described for example in "Ultrasound properties of lung tissue and their measurements" Ultrasound in Med. & Biol. Vol. 12, No. 6, pp. 483-499, 1986.

According to a preferred realisation of the invention, the catheters provided with sensors use as sensors electrodes and the physical property detected, which is depending by the density of the pulmonary tissue, is its bioelectric impedance. Moreover said electrodes, being inserted in the pulmonary tissue, can be used also to detect an electrocardiogram (ECG) without myoelectric artefacts and muscular tremors, ensuring an accurate control of the physical condition of the patient. The system for the measurement of the bioelectric impedance of the pulmonary tissue, can be a quadripolar or tripolar or bipolar system. The measurement of the bioelectric impedance of the pulmonary tissue may also occur in a pre-established temporal relation with the detected electrocardiogram or with the respiratory phases, to allow a comparison between similar pulmonary ventilation situations.

The presence in the container for the implantable device, of a ancillary sensor to detect the position of the patient, allows to recognise the modifications of the bioelectric impedance of the pulmonary tissue and/or of the ECG caused by the postural modifications of the patient.

According to an embodiment of the invention the intrapulmonary catheter provided with sensors electrodes which acquire the ECG and the detected physical property, depending upon the density of the pulmonary tissue, it is precisely the amplitude of the R wave of the acquired ECG, which was experimentally demonstrated to vary in the animal up to 50% of the base value, proportionally to the variations of the density of the pulmonary tissue.

The apparatus according to the invention can be used not only for diagnostic purposes, but also to guide and optimise the therapy of the heart diseases made with cardiac electric-stimulator and/or cardiac defibrillator and/or systems for the exhibition of drugs and/or for the drainage of the fluids accumulated in the body and/or for the mechanical circulatory assistance.

The present invention includes means for the communication with external devices by means of radio-frequency transmissions, also to make possible an automatic and remote control of the clinical situation of the patient.

The invention represents a remarkable improvement of the state of the art of the implantable devices for the therapy and the diagnosis of the heart diseases, because it allows, in one of the possible embodiments, the measurement of the specific bioelectric impedance of the pulmonary tissue, overcoming the limitations of the systems described until today, which by effecting a measurement of the transthoracic impedance with surface electrodes placed upon the skin or with electrodes placed inside the cardiac cavities, provide a measurement of the impedance determined prevalently by the variations of the volume of the heart and only partially by the contents of fluids of the pulmonary tissue, which will determine the density of the same.
Further features of the invention, and the advantages deriving therefrom, will appear better evident from the following description of a preferred embodiment of the same, made by way of non-limiting example, with reference to the figures of the attached sheet of drawings, in which:
- Figure 1 is a schematic view which shows the positioning in the human body of the implantable device with the intra-pulmonary catheters provided with sensors according to one of the solutions of the invention;
- Figures 2, 3 and 4 show with flow charts the realisations respectively with four, three and two electrodes for the measurement of the bioelectric impedance of the lung tissue of as much embodiments of the device according to the invention;
- Figures 2a, 2b and 2c show schematically as many different collocations of the electrodes in a quadripole system for the measurement of the electrical impedance;
- Figure 3a shows schematically the possible collocation of the electrodes in the tripolar for the measurement of the bioelectrical impedance;
- Figures 4a, 4b and 4c show schematically other possible collocations of the electrodes in a bipolar system for the measurement of the bioelectrical impedance;
- Figures 5 and 5a are graphics correlated between them which show respectively the variations of the bioelectric impedance of the lung tissue and of the left ventricular telediastolic pressure in an animal subjected to an overload of the hematic flow volume, by means of liquid infusion;
- Figure 6 is a graphic which shows an electrocardiogram registered between two different electrodes in the device according to the invention implanted in an animal;
- Figure 7 is a graphic which shows the variations of the bioelectric impedance caused by the respiration, registered by means of an intrapulmonary catheter device according to the invention;
- Figure 8 shows with flow charts a simplified realisation of the invention which provides the detecting of the electrocardiogram between two electrodes of the device and the measurement of the amplitude of the waves R;
- Figures 8a, 8b and 8c show schematically the possible collocations of the electrodes of the Figure 8;
- Figure 9 shows with flow charts a simplified realisation of the invention which provides the detecting of the bioelectric impedance as a difference between two impedance values obtained from two points of the pulmonary tissue, interested by the respective electrodes of the intrapulmonary catheter;
- Figure 9a shows schematically the possible collocation of the electrodes in the solution of Figure 9.

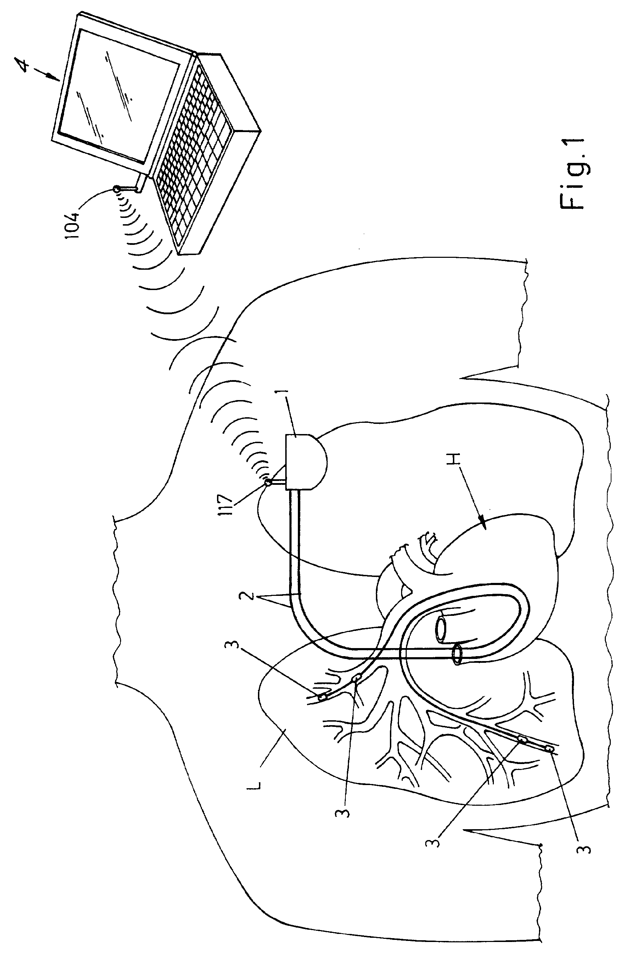

From Figure 1 which shows a possible embodiment of the device having therapeutics and/or diagnostics objectives according to the invention, it is noted that the same device is placed inside a implantable container 1, tight sealed and biologically inert, which can be itself electrically conductive in the use as electrode and to get involved in the measurement process to which the same device is predisposed. One or more intrapulmonary catheters 2 are electrically connected to the device placed inside the container 1 and by means of the intravenous access of the human body, normally used for the implant of standard stimulating electrical-catheters and therefore through the right portion of the heart H, they are inserted inside branches of the pulmonary artery of the patient. Preferably the intrapulmonary catheters 2 above mentioned, are placed in a same lung and preferably in the right lung L which is less involved by the volume of the heart, so that the work of the same catheters results less influenced by the movements effected by cardiac muscle during the systolic and diastolic phases.
In correspondence of the terminal end of the intrapulmonary catheters 2 are provided one or more electrical conductive electrodes 3 which are used to apply the measurement currents and/or to detect the corresponding potential differences proportional to the impedance of the pulmonary tissue and/or of the electrocardiogram.

The implantable device is pre-arranged in any suitable manner for the telemetric connection with an external programming and control device 4.

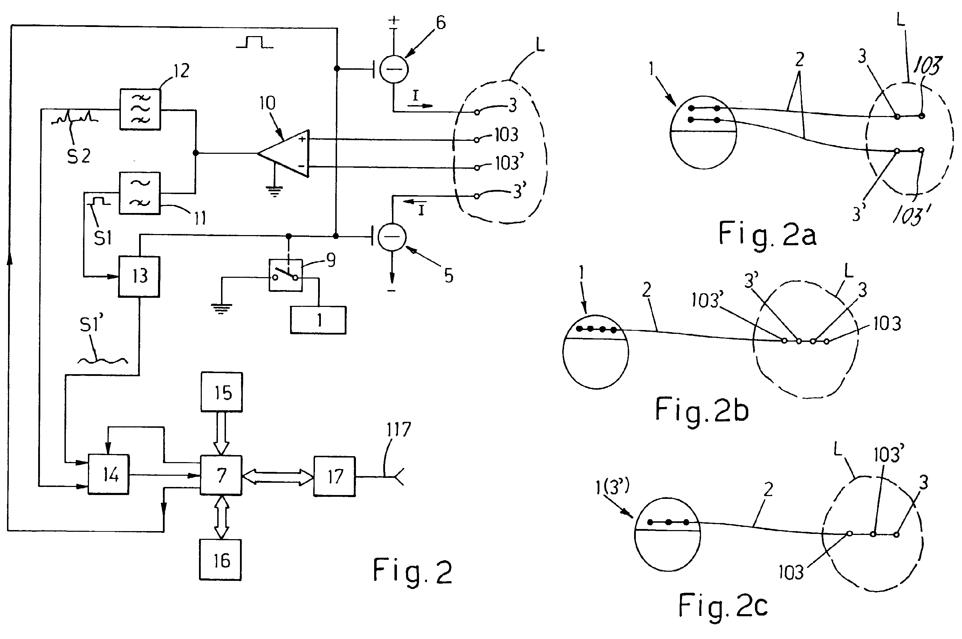

From Figure 2a it is noted that according to the embodiment shown in Figure 1, the device can be provided with a quadripole system of electrodes 3, placed for example in pairs upon the ends of two catheters 2, or placed with a correct distance between them upon a same catheter 2, as shown in Figure 2b. Figure 2c shows a further quadripole solution, with a single catheter 2 provided on the end with three electrodes suitably spaced between them and with the fourth electrode which is constituted by the electrically conductor body of the container 1 of the device. With numerals 3, 3' are indicated the emission electrodes for the measuring currents and with 103, 103' are shown the reception electrodes, to detect the corresponding differences of potential proportional to the bioelectric impedance and/or to detect the electrocardiogram.

From the flow chart of Figure 2, which shows the realisation details of the quadripole solution as from Figures 2a and 2b, it is noted that the current generators 5 and 6, under the time control of the microprocessor 7, generate an electric current of measure I, having a correct value, through the electrodes 3, 3' of the intrapulmonary catheters system above mentioned. During the formation of said current impulses, to allow the flow of the same current only through the electrodes 3, 3', by means of the block 9, the processor 7 temporarily disconnects the reference electrode constituted by the body of the container 1 of the device.
The reception electrodes 103, 103' are connected to a differential amplifier 10 which amplifies the differences of potential detected, proportional to the impedance of the pulmonary tissue and to the electrocardiographic signal. The output of the amplifier 10 is connected to the input of the high pass filter 11 and of the band pass filter 12, the first of which has a cut-off frequency of about 1 kHz and provides in output a signal S1 proportional to the bioelectric pulmonary impedance, while the band pass filter works preferably in frequency band comprised between 0,01 and 100 Hz and gives an output signal S2 which represents the electrocardiogram.
The outlet of the high pass filter 11 is connected to a sample and hold circuit 13 which samples the inlet signal and which supplies an analog signal S1' proportionate to the bioelectric impedance of the pulmonary tissue explored by the catheters 2.

The analog to digital converter 14, digitises the signal S1' and S2 for the processing unit 7 which receives the necessary algorithm for the various phases of the elaboration, by means of a ROM 15. The data elaborated by the unit 7 are stored in a RAM memory unit 16.

The telemetric unit 17, with its own antenna 117, ensures the necessary bi-directional communication with the antenna 104 of the external programming and enquiry unit 4 shown in Figure 1.

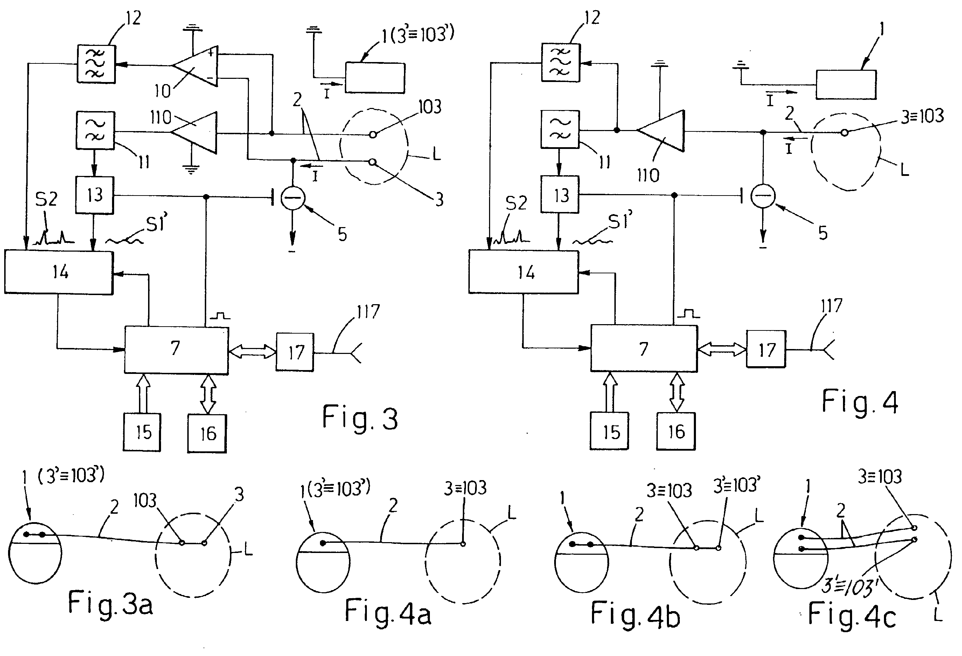

The solution shown in Figure 3a shows a tripolar measurement system which provides two electrodes 3 and 103 placed at the terminal end of a single intrapulmonary catheter 2 inserted in a branch of the pulmonary artery as from the Figure 1 and the electrodes 3 and 103 coinciding with a reference electrode, constituted by the electroconductive body of the container 1. From Figure 3 it is noticed that the microprocessor 7 drives the current generator 5 to feed the current impulses between the electrode 3 and the reference electrode constituted by the external body of the container 1, for the measurement of the bioelectric impedance of the portion of pulmonary tissue comprised between said electrode 3 of the intrapulmonary catheter 2 and the container 1. The difference of potential following to said current impulse and proportional to the bioelectric impedance is (measured) between the electrode 103 and the reference electrode constituted by the elettroconductive body of the container 1 and it is amplified by the amplifier 110 the output signal of which passes through the high pass filter 11 of the type above mentioned with reference to the Figure 2, which eliminates the low frequency signals, like the electrocardiographic signals. The outlet signal from the filter 11 is then sampled by the sample and hold unit 13 which is under the temporal control of the processor 7.

The electrocardiographic signal is then detected between the electrodes 3, 103, is amplified by means of the differential amplifier 10 and filtered by the band pass filter 12 which is similar to the one of Figure 2.

The following analog to digital conversion unit 14 digitises the signals relatives to the electrocardiogram S2 to the impedance S1 and transfers the same to the process unit 7 which, like in the previous case, is helped by the memories 15 and 16 and which is connected to the telemetric unit 17, with its own antenna 117.

The solution shown in Figure 4a is relative to a system for the bipolar measurement which provides the utilisation of a single intrapulmonary catheter 2 with a single electrode 3≡103 while the electrodes 3' and 103' coincide with the reference electrode constituted by the elettroconductive body of the container 1 of the implanted device which is referred to. The bipolar solution shown in Figure 4b provides the use of a single catheter 2 with two emitting electrodes 3, 3' suitably spaced at the ends, with the reception electrode 103 which coincides with 3 and with the electrode 103' which coincides with 3', while the bipolar solution according to Figure 4 provides the use of two catheters 2 with corresponding emission electrodes 3, 3' at the ends. Also in this case the reception electrode 103 coincides with 3 and the electrode 103' coincides with 3'.

From the flow chart of Figure 4, which shows the constructive details of the bipolar solution as from Figure 4a, it is noted that measurement electric current which derives from the current generator 5, is applied between the electrode 3 of the intrapulmonary catheter and the body of the container 1 and the corresponding difference of potential proportional to the bioelectric impedance, is measured between the same electrodes. In the same time, said electrodes are used to detect the electrocardiographic signal. The electrode 103≡3 is connected to a wide band amplifier 110 which amplifies both the signals correspondent to the bioelectric impedance and to the ECG. The output of the amplifier 4 is connected to a couple of high pass and band pass filters 11 and 12 similar to these of Figure 2 and the signal which comes out from the filter 11 is sampled by the unit 13 controlled by the processor 7.
The output analog signals from the unit 13 and from the filter 12 are digitised by the converter 14 and then processed by the processing unit 7 assisted by the memories 15 and 16 and connected to the telemetric unit 17 with its own antenna 117.

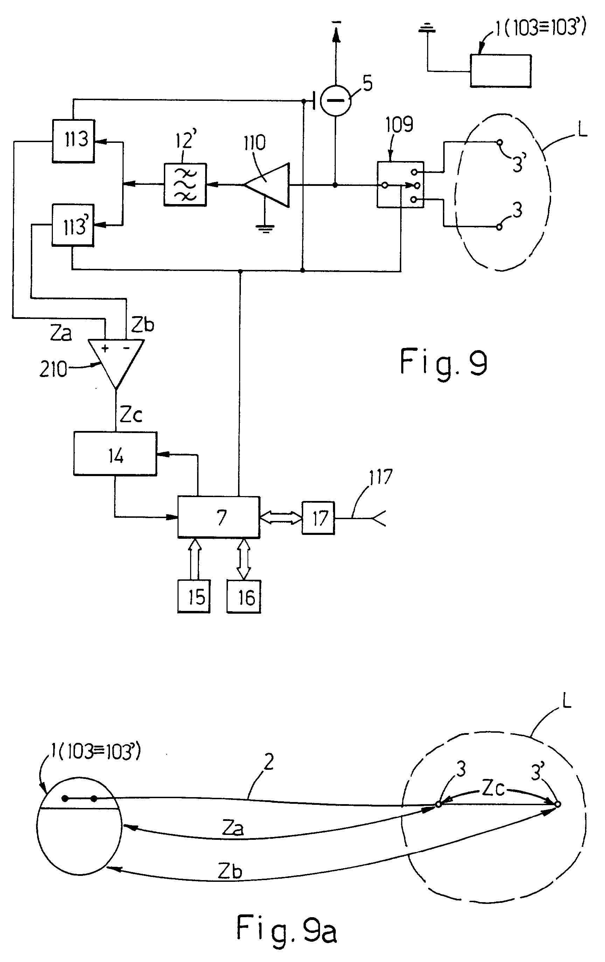

It is to be understood that the realisations illustrated with reference to the Figures 2, 3 and 4 are only some of the possible realisations of the device according to the invention and that other realisations must be considered protected by the same invention if adopt the same solution idea object of the invention. For example, a tripolar system as from Figure 9a, is suitable to detect the signals relative to the event to be mentioned, which are not influenced by contact resistors of the electrodes. The diagram of the Figure 9 relates to a solution as from Figure 9a, according to which the measurement currents produced by the generator 5 are alternatively applied, with a suitable temporal phase displacement determined by the static switch 109, between an electrode 3 of the intrapulmonary catheter 2 and the elettroconductive body 1 of the device and a further electrode 3' of the same catheter 2, opportunely displaced from said electrode 3, and the same body 1. The output signals from the sample and hold units 113, 113', which process the output signal from the band pass filter 12 which comes from the amplifier 110, correspond to the impedances Za and Zb measured between each of said electrodes and the body of the device and these same signals are provided in input to an amplifier 210 which gives an output signal Zc corresponding to the difference between the input signals and which is relative to the bioelectric impedance of the portion of pulmonary tissue explored by the said electrodes (see Figure 9a). Because the same measures of Za and Zb are influenced by the contact resistance of the electrodes, their difference (Za-Zb = Zc) will result relative to the pure bioelectrical impedance of the pulmonary tissue explored by the catheter of the device, without said contact resistances which cancel out reciprocally in said equation.

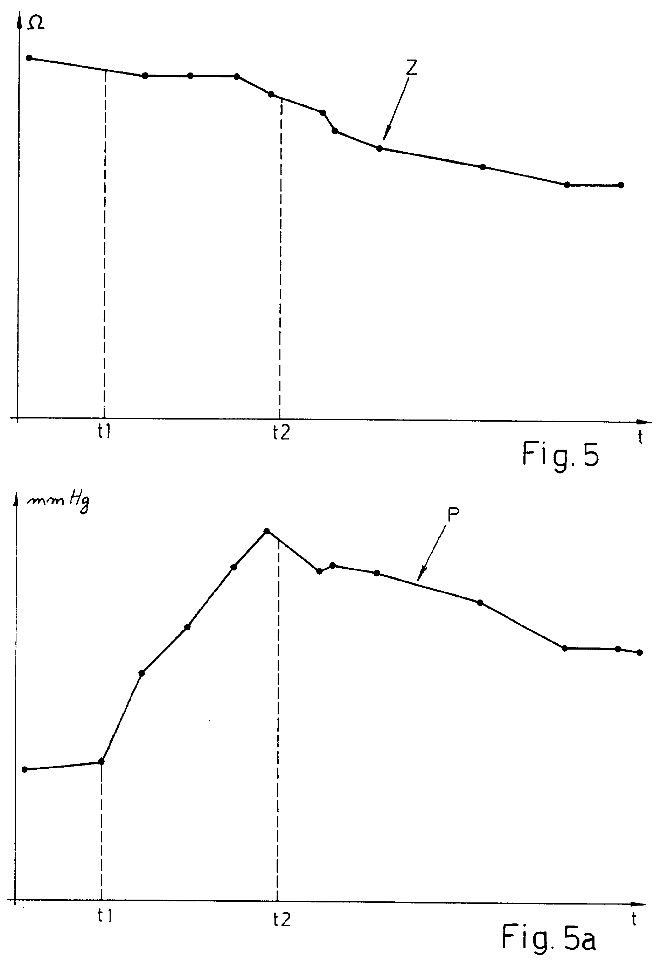

The curves of the Figures 5 and 5a show respectively the variations of the bioelectrical impedance Z in the pulmonary tissue, derived from an elaboration of the output signal from the block 13 and the variations of the telediastolic pressure P of the left ventricle, obtained with the usual pressure systems, in an animal exposed to a liquid overload by means of venous infusion of a glucosed solution which begins in the instant t1, and which ends in the instant t2 and which goes on few minutes. It can be noted how the haematic volume overload, caused by the infusion of liquids in the animal and documented by the variations of the left ventricular telediastolic pressure P, it is precisely detected by the variations of the bioelectrical impedance Z of the pulmonary tissue, which is reduced for the increase of the interstitial fluid.

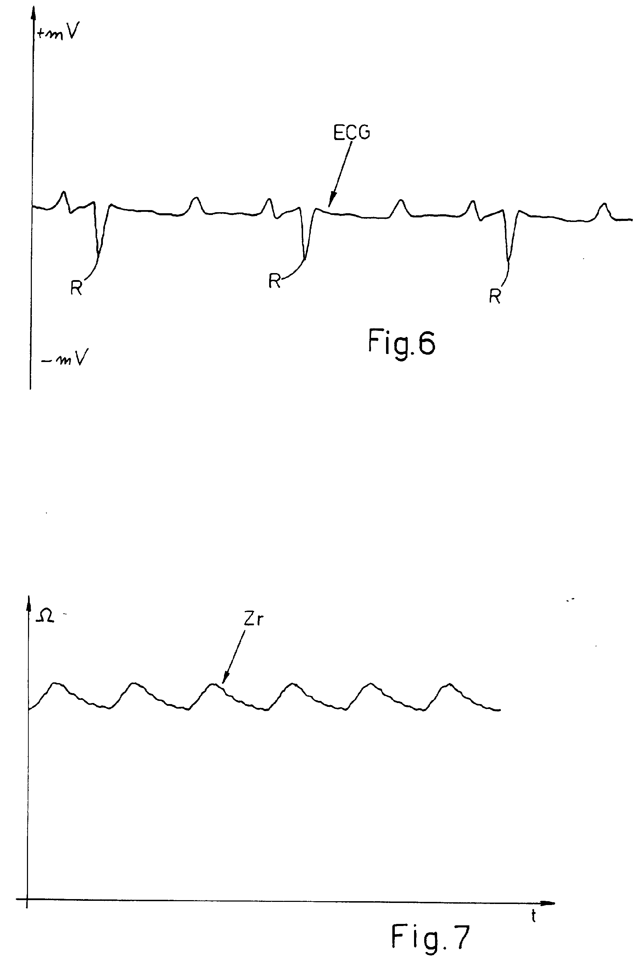

The diagram of Figure 6 shows an electrocardiographic tracing ECG as appears at the output of the band pass filter 12. It is evident the clearness of the ECG tracing which appears morphological similar to the one that can be obtained by means of a system with external electrodes.

The curve Zr shown in the diagram of Figure 7 is derivable from the output of the block 13 of Figure 2 and shows the bioelectrical impedance variations detectable at the output of the amplifier 10 of the solution of Figure 2, due to the respiration.

As mentioned in the introduction of the present description, from the analysis of the electrocardiogram obtained by means of the device is referred to, it is noted that the R waves of the same electrocardiogram ECG of Figure 6, tends to reduce its amplitude with a degree which is directly proportional to the increase of the fluid in the pulmonary tissue, until reach reduction of about 50% in the critical phase.

The device according to the invention can be pre-arranged with a more simplified solution respect to the solution described with reference to the Figures 2, 3 and 4 (see further) to detect by means of the catheter/catheters provided with sensors, the electrocardiographic signal and to detect the amplitude of the R waves of the signal of Figure 6.

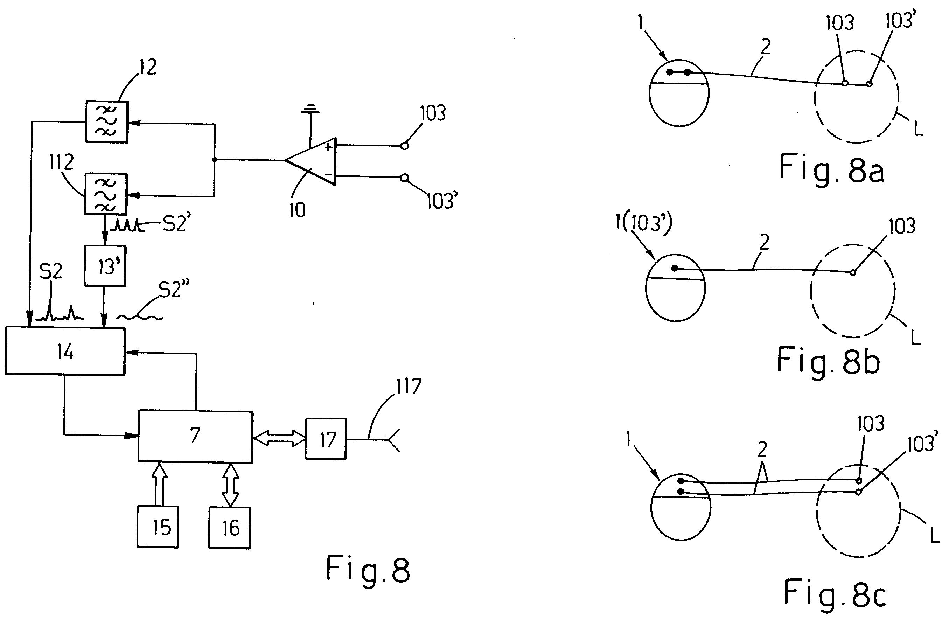

The solution shown in Figure 8a is relative to a system for the detection of the electrocardiogram and for the measurement of the amplitude of the waves R, which provides the utilisation of a single intrapulmonary catheter 2 with two electrodes 103 and 103' opportunely spaced between them at the ends. The solution shown in the Figure 8b provides on the other hand the utilisation of a single intrapulmonary catheter with an end electrode 103, while as electrode 103' is utilised the electroconductive body of the container 1. The solution of Figure 8c provides on the other end the utilisation of two intrapulmonary catheters 2 with corresponding electrodes 103 and 103' at the ends.

From the flow charts of figure 8, which shows the realisations details of a simplified solution of the invention for the detecting of the electrocardiogram and for the analysis of the amplitude of the waves R, as from Figures 8a, 8b and 8c, it is noted that the reception electrodes 103 and 103' are connected to the differential amplifier 10. The output of the amplifier 10 is connected to the input of the band pass filters 12 and 112, the first of which works preferably in a band of frequencies comprised between about 0,01 and 100 Hz and in output gives a signal S2 which represents the electrocardiogram, while the second band pass filter 112 works preferably in a frequency band which is comprised between about 10 and 60 Hz and which gives in output a signal S2' which represents the only R waves of the electrocardiogram. The output S2 of the band pass filter 112 is connected to a sample and hold circuit 13', which samples the input signal and which provides in output an analog signal S2", relative to the amplitude of the R waves of the electrocardiogram. The analog-to-digital converter 14 digitises the signals S and S" for the process unit 7, which receives the necessary algorithms for the various portions of elaboration, from the ROM memory 15. The data processed by the unit 17 are stored in the RAM memory unit 16. The telemetric unit 17, with its own antenna 117, ensures the required bi-directional communication with the antenna 104 of the programming and enquiry external unit 4 as shown in Figure 1.

It is to be understood that a same device cam be pre-arranged to detect both the variations of the bioelectrical impedance, both the variations of the amplitude of the R wave of the electrocardiogram obtained by means of the intrapulmonary catheter/s.

The process unit 7 of the several shown solutions, may be programmed to generate alarm signals if the measured quantities surpass the threshold values set out in the ROM 15 or from reference basic values and to activate the same device in its therapeutic and/or diagnostic functions. In case of alarm, the device can activate the exhibition to the patient of a suitable therapy, for example the activation of means for the electro-stimulation and/or for the electric defibrillation of the heart and/or the activation of means for the release of drugs and/or the activation of systems for the drainage of fluids accumulated in the body and/or means for the activation of means for the circulatory mechanic assistance of the heart.

It is to be understood that the description is referred to a preferred embodiment of the invention, to which can be brought several variations and modifications, which can be referred for example to the use of computation means or physical means, placed for example inside the container which achieve the same of the device, to detect the postural variation of the patient and to correlate with these the signal relative to the bioelectrical impedance and to the electrocardiographic signal.

## Claims

1. Implantable device for the measurement of the physical properties of the pulmonary tissue depending from its density, **characterised by** comprising:
- At least an intrapulmonary catheter (2) suitable to be inserted in a branch of the pulmonary artery;
- At least a sensor of density dependent physical properties of the pulmonary tissue, located at least inside said intrapulmonary catheter;
- Means for assessing the density dependent physical properties of the portion of pulmonary tissue explored by said intrapulmonary catheter provided with sensors (2);
- Means for processing the measure of the physical properties of the pulmonary tissue depending from its density and their variations, for diagnostic and/or therapeutic purposes, said measurement and elaboration means being placed inside an implantable container (1), to which is connected said catheter provided with sensors (2) and which contains the suitable means for the electric feeding.

2. Implantable device according to claim 1), in which the sensor for the density depending physical properties, comprises heat flow sensors, to detect the variations of the thermal conductivity of the pulmonary tissue when there is a variation in the fluid accumulation.

3. Implantable device according to claim 1) in which the sensor for the physical properties which are depending from the density of the pulmonary tissue, comprises optical sensors, to detect the transluminescence and/or colour variations of the pulmonary tissue at the variation of the fluid accumulation.

4. Implantable device according to claim 1), in which the sensor for the density dependent physical properties of the pulmonary tissue, comprises ultrasonic generators and relative means to detect the variations of the propagation velocity of the ultrasounds inside the pulmonary tissue, when there is a variation in the fluid accumulation.

5. Implantable device according to claim 1, in which the sensor/s of the density dependant physical properties of the pulmonary tissue comprises electrodes (3).

6. Implantable device according to claim 1) in which the body of the container (1) of the same device is realised in electroconductive material which can be used as electrode and which can be involved in the measurement process for which the same device is prefixed.

7. Implantable device according to claim 5) in which the means for the measurement of physical properties of the pulmonary tissue depending from its density, comprise means for the measurement, by means of said electrodes, the bioelectric impedance of the portion of pulmonary tissue explored by said catheter provided with sensors (2) and means for the processing of the obtained measure.

8. Implantable device according to claim 5), in which the means for the measurement of the physical properties of the pulmonary tissue depending from its density, comprise means to detect the electrocardiogram (ECG) by means of said electrodes, means for the measurement of the amplitude of the R waves of the detected ECG and means for the processing of the detected measure.

9. Implantable device according to claim 7) in which said means for the measurement of the bioelectric impedance, comprise also means to detect an electrocardiogram (ECG) by means of the electrodes of the same device and means for the processing of the detected ECG signal.

10. Implantable device according to claim 7), in which said means for the measurement of the bioelectric impedance of a portion of pulmonary tissue by means of said electrodes, comprises means to generate in said portions of tissue several measurement electric currents and means for the measurement of the differences of potential which are the consequence of the application of said measurement currents and which are proportional to the required bioelectric impedance.

11. Implantable device according to one or more of the preceding claims, in which the electrodes (1, 3) may be used, separated or in conjunction, with any suitable combination, for the application of said measurement electric currents and for the measurement of the differences of potential and of the bioelectric impedances which follows the application of said measurement currents.

12. Implantable device according to claim 10), in which said electric measurement currents are impulses and/or multiple and determined frequencies currents.

13. Implantable device according to claim 12, in which said measurement electric currents have components with frequencies comprise approximately between about 10 kHz and 1 MHz.

14. Implantable device according to claim 12), in which said means for the measurement of the differences of potential which follow to the application of said measurement currents and which are proportional to the required bioelectric impedances, comprise a differential amplifier (10), which amplifies the signals obtained between the electrodes of the same device, which are further filtered by a high-pass filter (11) and then processed by a sample and hold unit (13) and are finally digitised by means of a converter (14) before the transfer to the process unit (7).

15. Implantable device according to claim 14), in which the high-pass filter (11) works at about 1 kHz.

16. Implantable device according to claim 11), in which the measurement electric currents are applied between the two electrodes of the intrapulmonary catheter (2) and the differences of potential proportional to the bioelectric impedances are measured between the two same electrodes of the same catheter.

17. Implantable device according to claim 11) in which the measurement electric currents are applied between two electrodes of the intrapulmonary catheter and the differences of potential proportional to the bioelectric impedances are measured between two other electrodes of the same catheter.

18. Device according to claim 11), in which the measurement electric currents are applied between an electrode of a first intrapulmonary catheter and an electrode of a second intrapulmonary catheter and the differences of potential proportional to the bioelectric impedances are measured between another electrode of said first catheter and another electrode of the said second catheter.

19. Device according to claim 11), in which the measurement electric currents are applied between an electrode of a first intrapulmonary catheter and an electrode of a second intrapulmonary catheter and the differences of potential proportional to the bioelectric impedances, are measured between the same electrodes of the two said catheters.

20. Implantable device according to claim 11), in which the measurement electric currents are applied between an electrode of the intrapulmonary catheter (2) and the elettroconductive body of the same container (1) of the same device and the differences of potential proportional to the bioelectric impedances, are measured between another electrode of the same intrapulmonary catheter and the body of the container of the same device.

21. Implantable device according to claim 11), in which the measurement electric currents are applied between an electrode of an intrapulmonary catheter (2) and the elettroconductive body of the container (1) of the same device and the differences of potential proportional to the bioelectric impedances, are measured between the two other electrodes of the same intrapulmonary catheter.

22. Implantable device according to claim 11), in which the measurement electric currents are applied between an electrode of the intrapulmonary catheter (2) and the electroconductive body of the container (1) of the same device and the differences of potential proportional to the bioelectric impedances, are measured between the same electrode of the intrapulmonary catheter and the body of the container of the same device.

23. Implantable device according to claim 11), in which the measurement electric currents are applied between an electrode of the intrapulmonary catheter (2) and the elettroconductive body of the container (1) of the same device and between another electrode of the same intrapulmonary catheter, suitably spaced from the first electrode, and said body of the device, and the corresponding differences of potential proportional to the bioelectric impedances (Za, Zb) are measured disjointly between the same electrodes and the body of the device and means (210) are provided to obtain the bioelectric pulmonary impedance (Zc) as a difference between the two said obtained measures (Za, Zb).

24. Implantable device according to one or more of the preceding claims, in which the means for the detecting of the ECG comprise a differential amplifier (10) to amplify the signals detected by the measurement electrodes and comprise a band-pass filter (12) connected to the output of said differential amplifier.

25. Implantable device according to claim 24) in which the pass band filter (12) operates preferably between 0,01 and 100 Hz.

26. Implantable device according to claim 8), in which said means for the measurement of the amplitude of the R waves of the ECG comprise a differential amplifier (10) to amplify the signal detected by the measurement electrodes of the ECG, comprise a band-pass filter (112) which acts preferably between 10 and 60 Hz and comprise a sampling unit of the type sample and hold (13').

27. Implantable device according to one or more of the preceding claims, **characterised by** comprising a conversion unit (14) to digitise the analog output signals from the sample and hold (13, 13') and relative to the bioelectric impedance or to the R wave of the electrocardiographic signal and in output from the band-pass filter (12) which provides the electrocardiographic signal (ECG), the output of said conversion unit being connected to a processing unit (7) which provides the required temporal control to said sampling unit (13) and to the converter (14), which is assisted by a ROM (15) and RAM (16) memory unit which provide respectively the processing algorithms and which register the measured and processed data, comprehensive of the possible identification of alarm situations, said processing unit being connected to a break-in radio-frequency unit (17) with its antenna (117).

28. Implantable device according to claim 1) **characterised by** comprising external radio-frequency means (4, 104) by means of which the same device can be enquired and can be programmed for the diagnostic and/or therapeutic functions, also with the predisposition of alarm thresholds.

29. Implantable device according to claim 6), in which the processing means for the detected bioelectric impedance, comprise any mathematics elaboration means of the signal to detect at least the minimum and maximum values, the peak to peak amplitude, the medium value, the period, the derivate and the finite integral.

30. Implantable device according to one or more of the preceding claims, in which the processing means of the electrocardiogram (ECG) comprise any mathematics elaboration means of the signal to detect at least the minimum and maximum values, the peak to peak amplitude, the medium value, the period, the derivate and the finite integral.

31. Implantable device according to one or more of the preceding claims, in which said processing means comprise means to measure the bioelectric impedance in a pre-determined temporal relation with the electrocardiographic signal (ECG).

32. Implantable device according to one or more of the preceding claims, in which said processing means comprise means to determine the respiratory parameters, among which the respiratory frequency, the speed and the volume of the respiration and the ventilation per minute.

33. Implantable device according to claim 32), in which said processing means comprise means for the analysis of the bioelectric impedance in correspondence of pre-determined temporal phases of said respiratory parameters.

34. Implantable device according to one or more of the preceding claims, in which the processing means comprise any calculating means to detect the posture variations of the patient in relation to the bioelectric impedance variations.

35. Implantable device according to one or more of the preceding claims, in which said processing means comprise any calculating means to detect the posture variations of the patient in relation to the ECG variations.

36. Implantable device according to one or more of the preceding claims, in which said processing means comprise means which detect the posture variations of the patient in relation to the variations of the ECG and in function of the bioelectric impedance variations.

37. Implantable device according to one or more of the preceding claims, in which the processing means of the ECG comprise calculating means to detect the normal or pathologic of the heart due to heart failure, ischemia or arrhythmia.

38. Implantable device according to claim 1) **characterised by** comprising a postural sensor inside of the container (1) of the same device.

39. Implantable device according to one or more of the preceding claims, in which said processing means comprise means to detect the variation of the bioelectric impedance in function of the posture variations.

40. Implantable device according to one or more of the preceding claims, in which said processing means comprise means to detect the electrocardiogram (ECG) variations in function of the posture variations.

41. Device according to one or more of the preceding claims, **characterised by** comprising means to verify if the measured physical property depending from the density of the pulmonary tissue exceeds programmed threshold values or basal reference values and to generate consequent alarm signals, to activate the same device in the therapeutic and/or diagnostic function.

42. Implantable device according to claim 1), **characterised by** comprising means which in function of the measured physical properties, which depend from the density of the pulmonary tissue, and in function of the variations of said physical properties, provide to exhibit to the patient a pre-established therapy.

43. Implantable device according to claim 42), in which said therapy comprises the activation of means for the electrostimulation of the heart.

44. Implantable device according to claim 42), in which said therapy comprises the activation of means for the electric defibrillation of the heart.

45. Implantable device according to claim 42), in which said therapy comprises the activation of systems for the releasing of drugs.

46. Implantable device according to claim 42), in which said therapy comprises the activation of systems for the drainage of fluids accumulated in the body.

47. Implantable device according to claim 42), in which said therapy comprises the activation of means for the mechanical circulatory assistance to the heart.

48. Implantable device according to claim 42), in which said therapy comprises the activation of means for the stimulation and/or the electric defibrillation of the heart and/or for the exhibition of drugs and/or for the drainage of the fluids accumulated in the body and/or for the mechanical circulatory assistance to the heart.
